# EUROPÄISCHE PATENTSCHRIFT

(11) **EP 0 331 769 B1**
(45) Veröffentlichungstag und Bekanntmachung des Hinweises auf die Patenterteilung: **04.08.1993**
(21) Anmeldenummer: 88103522.4
(22) Anmeldetag: 07.03.1988
(51) Int. Cl.: A61B 5/103, G01G 19/44, G01L 1/00

(54) **Vorrichtung zum Messen der Ortsaufgelösten Druckverteilung**
Device for measuring bidimensional pressure distribution
Dispositif de mesure de la répartition bidimensionnelle de la pression

(43) Veröffentlichungstag der Anmeldung: 13.09.1989
(73) Patentinhaber: Brunner, Wolfgang, D-88305 Isny (DE); von Zech, Ludwig, D-88316 Isny (DE)
(72) Erfinder: Brunner, Wolfgang, D-88305 Isny (DE); von Zech, Ludwig, D-88316 Isny (DE)
(74) Vertreter: Säger, Manfred, Dipl.-Ing.

(56) Entgegenhaltungen:
- EP-A- 0 143 046
- EP-A- 0 230 315
- DE-A- 2 529 475
- FR-A- 2 513 508
- US-A- 3 894 437
- US-A- 3 906 931
- US-A- 4 426 884

## Beschreibung

Die Erfindung betrifft eine Vorrichtung gemäß dem Oberbegriff des Hauptanspruches.

Die US-A-3 894 437 beschreibt ein Verfahren und eine Vorrichtung zur dynamischen Analyse der Haltung, z.B. von Menschen, beispielsweise zur Diagnose von körperlichen Gebrechen. Die Vorrichtung enthält eine Plattform, an der zumindest ein Fühler für das Maß der Scherung der Plattform vorgesehen ist.

Eine gattungsgemäße Vorrichtung ist aus der DE-A 25 29 475 bekannt und dient zum ortsaufgelösten Messen der Druckverteilung, beispielsweise des Fusses beim Abrollvorgang. mit dieser bekannten Vorrichtung, ist es möglich, nur orthogonale Kräfte zur Ober- und Unterseite der Plattform zu messen.

Der Erfindung liegt deshalb die Aufgabe zugrunde, eine Vorrichtung zum ortsauf gelösten Messen der Druckresteilung gemäß dem Oberbegriff so weiterzubilden, daß auch Kräfte in der Ebene der Plattform gemessen werden können.

Diese Aufgabe wird bei einer gattungsgemäßen Vorrichtung gemäß dem Oberbegriff des Hauptanspruches erfindungsgemäß durch dessen kennzeichnende Merkmale gelöst.

Bei Verwendung von vorzugsweise drei Fühlern, die entweder Weg- oder Kraftfühler sein können, können alle Scherkräfte in der Ebene, auch Drehbewegungen erfaßt werden. Infolge der erfindungsgemäßen Ausbildung kann die Plattform dünn sein, da eine Vielzahl von nichtkomprimierbaren, jedoch scherbaren Lagerstellen vorhanden sind, die die gesamte Unterseite der Plattform und damit auch diese abstützen. Es wird durch diese Ausbildung ein niedriger Aufbau erreicht, wodurch auch die Masse der Plattform selbst gering gehalten werden kann. Infolge geringerer Masse der Platte ergeben sich auch erzielbare höhere Meßgenauigkeiten. Im übrigen ist die Vorrichtung einfach und daher kostengünstig herzustellen, sowie robust.

Zweckmäßige Ausgestaltungen und Weiterbildungen der Erfindung sind in den Unteransprüchen gekennzeichnet.

Ein bevorzugtes Ausführungsbeispiel der Erfindung wird nachfolgend unter Bezugnahme auf die Zeichnung näher erläutert. In dieser zeigt:
- Figur 1: die Vorrichtung im teilweise abgebrochenen Querschnitt und
- Figur 2: die Einzelheit II gemäß Fig.1.

Die insgesamt mit 5 bezeichnete Vorrichtung weist zum Messen der ortsaufgelösten Druckverteilung des Fusses eines Menschen eine Plattform 6 mit einer ebenen Oberseite 7 und einer ebenfalls ebenen Unterseite auf. Diese Plattform 6 ist an sich bekannt.

Zur Messung von Scherkräften an der Plattform ist unter dieser eine ortsfeste Grundplatte 8 angeordnet, wobei zwischen der Plattform 6 und der Grundplatte 8 ein Zwischenbereich 9 angeordnet ist.

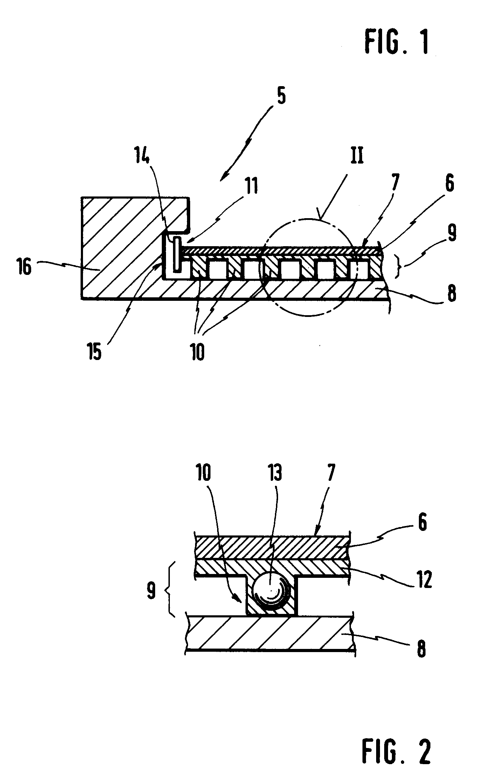

Der Zwischenbereich 9 weist eine Vielzahl von scherbaren und praktisch nicht komprimierbaren Auflagerstellen 10 für die Plattform 6 auf und dient überdies als elastisches Rückstellglied. Der Zwischenbereich 9 ist, wie Fig. 2 zeigt, in Form einer flächig durchgehenden Schicht 12 auf der Unterseite der Plattform 6 angeordnet, über die die Auflagerstellen 10 nach unten vorspringen. Die Auflagerstellen 10 können, wie Fig. 2 schematisch ebenfalls zeigt, im Inneren eine Kugel 13, vorzugsweise aus Stahl aufweisen, die in etwa in ihrem Durchmesser der Höhe der Auflagerstelle 10 entspricht. Der Zwischenbereich besteht vorzugsweise aus Kunststoff, insbesondere Gummi oder Silikonkautschuk.

Ferner sind zumindest drei Fühler 11 (Fig. 1) zur Feststellung des Maß der Scherung der Plattform 6 gegenüber der Grundplatte 8 vorgesehen. Beim wiedergegebenen Ausführungsbeispiel ist der Fühler als Wegfühler kapazitiv in Form einer senkrecht zur Oberseite 7 der Plattform 6 verlaufenden Kondensatorplatte 14 ausgebildet, der die Wand 15 des hochgezogenen Randbereiches 16 der Grundplatte 8 parallel gegenüberliegt. Durch Veränderung des Abstandes der Kondensatorplatte 14 von der Wand 15 ändert sich auch die Kapazität, die gemessen wird. Die elektrischen Zuleitungen zu der Kondensatorplatte 14 sind aus Gründen der Übersichtlichkeit in der Zeichnung nicht dargestellt.

## Patentansprüche

1. Vorrichtung zur Messung von mechanischen Kraftverteilungen auf der Basis matrixförmig angeordneter, sequentiell abfragbarer Meßfühler beliebiger Anzahl, welche durch die Krafteinwirkung proportionale elektrische Signale abgeben, zum Messen der ortsaufgelösten Druckverteilung des Fußes beim Abrollen mit einer auf der Ober- und Unterseite ebenen Plattform,
**dadurch gekennzeichnet**,
daß zur Messung von Scherkräften an der Plattform (6) unter dieser eine ortsfeste Grundplatte (8) mit einem auf deren Oberseite festgelegten Zwischenbereich (9) angeordnet ist, daß der Zwischenbereich eine Vielzahl von nicht-komprimierbaren, jedoch scherbaren Auflagerstellen (10) für die Plattform (6) aufweist und für diese als elastisches Rückstellglied dient und daß zumindest ein Fühler (11) für das Maß der Scherung der Plattform (6) gegenüber der Grundplatte (8) vorgesehen ist.

2. Vorrichtung nach Anspruch 1, **dadurch gekennzeichnet,** daß die Auflagerstelle zylinderförmig ausgebildet sind.

3. Vorrichtung nach Anspruch 1 oder 2, **dadurch gekennzeichnet**, daß die Auflagerstellen (10) vorzugsweise kreiszylindrisch ausgebildet sind.

4. Vorrichtung nach Anspruch 3, **dadurch gekennzeichnet,** daß der Zwischenbereich (9) zumindest eine flächig durchgehende Schicht (12) aufweist, über die die Auflagerstellen (10) vorstehen.

5. Vorrichtung nach einem der Ansprüche 1 bis 4, **dadurch gekennzeichnet,** daß der Zwischenbereich (9) zwei durchgehende Schichten aufweist, die über die Auflagerstellen (10) miteinander verbunden sind.

6. Vorrichtung nach einem der Ansprüche 1 bis 5, **dadurch gekennzeichnet**, daß der Zwischenbereich (9) aus Kunststoff besteht.

7. Vorrichtung nach Anspruch 6, **dadurch gekennzeichnet**, daß der Zwischenbereich aus Gummi oder Silikonkautschuk besteht.

8. Vorrichtung nach einem der Ansprüche 1 bis 7, **dadurch gekennzeichnet**, daß die Auflagerstellen (10) als scherbare, gleichmäßig verteilte Noppen ausgebildet sind.

9. Vorrichtung nach einem der Ansprüche 1 bis 8, **dadurch gekennzeichnet**, daß die Auflagerstellen (10) im Inneren Kugeln (13), vorzugsweise aus Stahl aufweisen.

10. Vorrichtung nach Anspruch 9, **dadurch gekennzeichnet**, daß die kreiszylindrischen Lager stellen mit den eingesetzten Kugeln (13) eine Höhe von 0,8- bis 1,5-fachen des Durchmessers der insgesamt gleichgroßen Kugeln (13) aufweist.

11. Vorrichtung nach einem der Ansprüche 1 bis 10, **dadurch gekennzeichnet,** daß zur Messung aller Scherkräfte und Drehmomente drei Fühler (11) vorgesehen sind.

12. Vorrichtung nach einem der Ansprüche 1 bis 11, **dadurch gekennzeichnet,** daß die Fühler (11) kapazitiv wirken.

13. Vorrichtung nach Anspruch 12, **dadurch gekennzeichnet**, daß der Fühler (11) eine senkrecht zur Oberseite (7) der Plattform (6) laufende Kondensatorplatte (14) aufweist und daß parallel zu dieser eine hochreichende Wand (15) der Grundplatte (8) gegenüberliegt.

## Claims

1. Apparatus for the measurement of mechanical force distributions on the basis of any number of sequentially interrogatable measurement probes arranged in a matrix, which emit proportional electrical signals due to the force effect, for measuring the locally dispersed pressure distribution of the foot during the rolling motion with a level platform on the top and bottom sides, characterised in that for the measurement of shear forces on the platform (6) there is arranged below the latter a fixed base plate (8) with an intermediate region (9) fixed to its top side, that the intermediate region comprises a plurality of non-compressible, but shearable support points (10) for the platform (6) and acts as a flexible restoring member therefor and that at least one probe (11) is provided for the degree of the shear of the platform (6) relative to the base plate (8).

2. Apparatus according to claim 1, characterised in that the support points are cylindrical in shape.

3. Apparatus according to claim 1 or 2, characterised in that the support points (10) are preferably circular Cylindrical in shape.

4. Apparatus according to claim 3, characterised in that the intermediate region (9) comprises at least one two-dimensionally continuous layer (12) beyond which the support points (10) project.

5. Apparatus according to any one of claims 1 to 4, characterised in that the intermediate region (9) comprises two continuous layers which are connected to one another via the support points (10).

6. Apparatus according to any one of claims 1 to 5, characterised in that the intermediate region (9) is made of plastics material.

7. Apparatus according to claim 6, characterised in that the intermediate region is made of rubber or silicone rubber.

8. Apparatus according to any one of claims 1 to 7, characterised in that the support points are formed as shearable, uniformly distributed burls.

9. Apparatus according to any one of claims 1 to 8, characterised in that the support points (10) comprise on the inside balls (13), preferably of steel.

10. Apparatus according to claim 9, characterised in that the circular cylindrical bearing points with the inserted balls (13) have a height of 0.8 to 1.5 times the diameter of the generally equal-sized balls (13).

11. Apparatus according to any one of claims 1 to 10, characterised in that for measuring all the shear forces and torques three probes (11) are provided.

12. Apparatus according to any one of claims 1 to 11, characterised in that the probes (11) operate capacitively.

13. Apparatus according to claim 12, characterised in that the probe (11) comprises a capacitor plate (14) extending perpendicular to the top side (7) of the platform (6) and that parallel with and opposed thereto there is located a vertical wall (15) of the base plate (8).

## Revendications

1. Dispositif pour mesurer des distributions de forces mécaniques à l'aide de palpeurs de mesure, disposés en un nombre quelconque en forme matricielle et pouvant être interrogés séquentiellement, lesquels palpeurs, sous l'effet des forces exercées, émettent des signaux électriques proportionnels permettant de mesurer la distribution des pressions localisées du pied se déroulant sur une plateforme, plane sur sa face supérieure et sa face inférieure, **CARACTERISE:**
- en ce que, pour mesurer des forces de cisaillement de la plateforme (6), sous cette dernière est disposée une plaque de base fixe (8) comportant, fixée sur sa face supérieure, une zone intermédiaire (9),
- en ce que la dite zone intermédiaire présente une pluralité de points d'appui (10) ne pouvant pas être comprimés mais pouvant subir les forces de cisaillement de la plateforme (6) et servir d'élément de rappel élastique de cette dernière et,
- en ce qu'au moins un palpeur (11) est prévu pour, par référence à la plaque de base (8), mesurer le cisaillement de la plateforme (6).

2. Dispositif selon la revendication 1 **caractérisé** en ce que le point d'appui est réalisé en forme de cylindre.

3. Dispositif selon la revendication 1 ou 2 **caractérisé** en ce que les points d'appui (10) sont réalisés de préférence sous forme de cylindres.

4. Dispositif selon la revendication 3 **caractérisé** en ce que la zone intermédiaire (9) présente au moins une couche (12) continue plane sur laquelle font saillie les points d'appui (10).

5. Dispositif selon l'une des revendications 1 à 4 **caractérisé** en ce que la zone intermédiaire présente deux couches continues qui sont reliées mutuellement par les points d'appui (10).

6. Dispositif selon l'une des revendications 1 à 5 **caractérisé** en ce que la zone intermédiaire est constituée en matière plastique.

7. Dispositif selon la revendication 6 **caractérisé** en ce que la zone intermédiaire est constituée en caoutchouc ou en caoutchouc au silicone.

8. Dispositif selon l'une des revendications 1 à 7 **caractérisé** en ce que les points d'appui (10) sont réalisés sous forme de boutons régulièrement distribués et pouvant être cisaillés.

9. Dispositif selon l'une des revendications 1 à 8 **caractérisé** en ce que les points d'appui (10) présentent, à l'intérieur, des billes (13), de préférence, en acier.

10. Dispositif selon la revendication 9 **caractérisé** en ce que les points d'appui cylindriques à l'intérieur desquels se trouvent les billes (13) présentent une hauteur comprise de 0,8 à 1,5 fois le diamètre des billes (13) toutes de même dimension.

11. Dispositif selon l'une des revendications 1 à 10 **caractérisé** en ce que trois palpeurs (11) sont prévus pour la mesure de toutes les forces de cisaillement et de tous les couples.

12. Dispositif selon l'une des revendications 1 à 11 **caractérisé** en ce que les palpeurs (11) ont un effet capacitif.

13. Dispositif selon la revendication 12 **caractérisé** en ce que le palpeur (11) présente une plaque de condensateur (14) s'étendant perpendiculairement à la face supérieure (7) de la plateforme (6) et en ce que parallèlement à cette plaque de condensateur (14), une paroi (15) de hauteur importante fait face à la plaque de base (8).
